# EUROPÄISCHE PATENTSCHRIFT

(11) **EP 1 858 400 B1**
(45) Veröffentlichungstag und Bekanntmachung des Hinweises auf die Patenterteilung: **30.03.2016**
(21) Anmeldenummer: 06703858.8
(22) Anmeldetag: 21.01.2006
(51) Int. Cl.: A61B 5/00

(54) **SENSORSYSTEM ZUR MESSUNG, ÜBERTRAGUNG, VERARBEITUNG UND DARSTELLUNG VON PHYSIOLOGISCHEN PARAMETERN**
SENSOR SYSTEM FOR MEASURING, TRANSMITTING, PROCESSING AND DISPLAYING PHYSIOLOGICAL PARAMETERS
SYSTEME DE DETECTION POUR LA MESURE, LA TRANSMISSION, LE TRAITEMENT ET L'AFFICHAGE DE PARAMETRES PHYSIOLOGIQUES

(30) Priorität: 25.02.2005 DE 102005008627
(43) Veröffentlichungstag der Anmeldung: 28.11.2007
(73) Patentinhaber: RAUMEDIC AG, 95233 Helmbrechts (DE)
(72) Erfinder: REICHENBERGER, Robert, 95632 Wunsiedel (DE); KUNZE, Gerd, 08297 Zwönitz (DE); GÖHLER, Karl-Heinz, 08279 Zwönitz (DE)
(74) Vertreter: Hofmann, Matthias
(86) Internationale Anmeldenummer: PCT/EP2006/000526
(87) Internationale Veröffentlichungsnummer: WO 2006/089606

(56) Entgegenhaltungen:
- US-A1- 2004 122 297
- US-B1- 6 694 180

## Beschreibung

Die Erfindung betrifft ein Sensorsystem zur Messung, Übertragung, Verarbeitung und Darstellung von physiologischen Parametern wie in den Ansprüchen definiert.

Ein derartiges Sensorsystem ist bekannt aus der US 2004/0122297 A1.
Dort sind einerseits Sensorsysteme beschrieben, die mit kabelgebundenen Sensoren arbeiten und andererseits Sensorsysteme, die mit kabellosen Sensoren arbeiten.

Aus der US 6 694 180 B1 ist ein Sensorsystem mit mehreren Telemetrie-Sensoren bekannt.

Ein weiteres Sensorsystem nach dem Oberbegriff des Anspruchs 1 ist durch offenkundige Vorbenutzung bekannt. Bei der Anwendung dieses bekannten Sensorsystems wird der Patient mit entsprechenden Sensoren verbunden, die über Kabel an das Datenverarbeitungsmodul angeschlossen werden. Es ist dann möglich, die physiologischen Parameter des Patienten zu messen, zu übertragen, zu verarbeiten und darzustellen. Nachteilig ist, dass der Patient hierbei stationär überwacht werden muss.

Es ist daher eine Aufgabe der vorliegenden Erfindung, ein Sensorsystem der eingangs genannten Art derart weiterzubilden, dass dessen Flexibilität erhöht und insbesondere die Bewegungsfreiheit des Patienten erhöht wird.

Diese Aufgabe ist erfindungsgemäß gelöst durch ein Sensorsystem mit den im Kennzeichnungsteil des Anspruchs 1 angegebenen Merkmalen.

Erfindungsgemäß wurde erkannt, dass bereits vorbekannte Grund-Sensorsysteme, die mit kabelgebundenen Sensoren arbeiten, für die Zusammenarbeit mit nicht kabelgebundenen Telemetrie-Sensoren angepasst werden können, wenn entsprechende Erweiterungskomponenten vorgesehen werden. Dabei wird der Umstand genutzt, dass das Datenverarbeitungsmodul der vorbekannten, kabelgebundenen Sensorsysteme in der Regel eine Schnittstelle zur externen Datenkommunikation aufweist. Bei dieser Schnittstelle handelt es sich in den meisten Fällen um eine Standard-Schnittstelle. Das Erweiterungsmodul stellt nun die Möglichkeit zur Verfügung, das vorbekannte Grund-Sensorsystem für die Kommunikation mit an sich zum Beispiel aus der WO 02/062 215 A2 bereits bekannten Telemetrie-Sensoren zu erweitern. Ohne dass tiefgreifende Änderungen in der Datenkommunikation des vorbekannten Sensorsystems erforderlich sind, können durch die Verwendung des zusätzlichen Erweiterungsmoduls nun auch die Daten von Telemetrie-Sensoren gemessen, übertragen, verarbeitet und dargestellt werden. Damit ist es möglich, ausschließlich Daten kabelloser Telemetrie-Sensoren zu verarbeiten, ohne dass dabei auf die bewährten Komponenten "Datenverarbeitungsmodul" und "Darstellungseinrichtung" verzichtet werden muss. Mit Hilfe des Erweiterungsmoduls wird dem Datenverarbeitungsmodul durch eine entsprechende Emulation einfach ein zusätzlicher kabelgebundener Sensor vorgespiegelt. In diesem Fall ist überhaupt kein Eingriff in die Hard- beziehungsweise Software des vorbekannten Sensorsystems erforderlich. Das Sensorsystem ist daher sehr flexibel einsetzbar.

Ein Erweiterungsmodul nach Anspruch 2 kann dem Datenverarbeitungsmodul Arbeit abnehmen, sodass die Verarbeitung im Datenverarbeitungsmodul schneller und effizienter erfolgt. Alternativ ist es möglich, das Erweiterungsmodul als reines Übertragungsmodul ohne zwischengeschaltete Datenverarbeitung zu realisieren, sodass die vollständige Datenverarbeitung im Datenverarbeitungsmodul erfolgt.

Eine Unterteilung des Erweiterungsmoduls nach Anspruch 3 erlaubt zum Beispiel ein Auslesen von Telemetrie-Sensoren mit Hilfe der Leseeinheit direkt am Körper des Patienten, sodass die Sensor-Funkmodule der Telemetriestrecken vorteilhaft niedrige Leistungen haben können. Einlesen und Übertragen der Telemetrie-Sensordaten können dann flexibel an verschiedenen Orten stattfinden.

Beim Erweiterungsmodul nach Anspruch 4 ist die Flexibilität nochmals vorteilhaft erhöht. Es ist beispielsweise möglich, dass der Patient eine Leseeinheit mit sich führt, während er seiner normalen Alltagsbeschäftigung nachgeht. Die Leseeinheit kann dann die ausgelesenen Telemetrie-Sensordaten über die kabellose Telemetriestrecke an die Übertragungseinheit weiterleiten. Dies kann zum Beispiel über das Internet erfolgen.

Eine bidircktionale Signalverbindung nach Anspruch 5 erweitert die Anwendungs möglichkeiten des Sensorsystems betröcht lich

Eine Steuereinheit nach Anspruch 6 erlaubt eine Sensorsteuerung oder -regelung durch das Datenverarbeitungsmodul beziehungsweise das Erweiterungsmodul. Die Sensoren, das heißt der mindestens eine kabelgebundene Sensor und/oder der mindestens eine Telemetrie-Sensor, können dann zum Beispiel initialisiert, in ihrer Betriebsweise verändert und/oder an sich verändernde Umgebungsveränderungen angepasst werden.

Sensoren zur Messung der physiologischen Parameter, die im Anspruch 7 aufgelistet sind, können vorteilhaft beim Sensorsystem eingesetzt werden. Es ergibt sich eine effektive Überwachung von Patientenparametern in unterschiedlichen Diagnose- und Therapiesituationen.

Mindestens ein externer Sensor nach Anspruch 8 ist zur Kalibrierung des Sensorsystems besonders geeignet.

Eine Anordnung des externen Sensors nach Anspruch 9 führt zu einem kompakten Sensorsystem.

Eine Telemetrie-Schnittstelle nach Anspruch 10 hat sich als für den Einsatz im Sensorsystem vorteilhafter Standard herausgestellt.

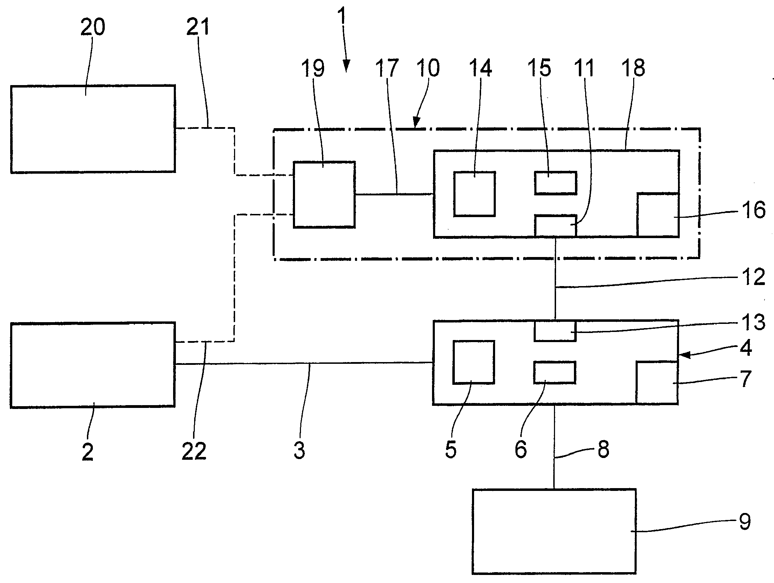

Ein Ausführungsbeispiel der Erfindung wird nachfolgend anhand der Zeichnung näher erläutert. Die einzige Figur zeigt schematisch ein Sensorsystem zur Messung, Übertragung, Verarbeitung und Darstellung von physiologischen Parametern.

Das in der Zeichnung insgesamt mit 1 bezeichnete Sensorsystem umfasst einen Sensor 2 zur Messung mindestens eines physiologischen Parameters. Beispiele derartiger Sensoren sind beschrieben in der DE 102 39 743 A1 sowie in der WO 02/062 215 A2. Mit dem Sensor 2 kann mindestens einer der nachfolgend aufgelisteten physiologischen Parameter gemessen werden: Hirndruck, Sauerstoffgehalt von Körperflüssigkeit beziehungsweise -gewebe, CO₂-Gehalt von Körperflüssigkeit beziehungsweise -gewebe, pH-Wert von Körperflüssigkeit, Körpertemperatur, Blutzuckergehalt, Blutfluss. Der Sensor 2 ist zur Messung dieses mindestens einen Parameters mit dem Kopf beziehungsweise Körper des Patienten verbunden beziehungsweise ist in diesen implantiert. Auch mehrere Sensoren 2 können beim Sensorsystem 1 vorgesehen sein.

Über ein Signalkabel 3 steht der Sensor 2 mit einem Datenverarbeitungsmodul 4 in Signalverbindung. Die Signalverbindung über das Signalkabel 3 ist bidirektional ausgeführt. Das Datenverarbeitungsmodul 4 dient zum Einlesen der Sensordaten und zum Verarbeiten von diesen, das heißt insbesondere zum Aufzeichnen, Speichern und Auswerten der Sensordaten. Zudem kann mit Hilfe des Datenverarbeitungsmoduls 4 der Sensor 2 gesteuert beziehungsweise geregelt werden. Hierzu weist das Datenverarbeitungsmodul 4 eine integrierte Steuereinheit 5 auf. Zur Verarbeitung der vom Sensor 2 empfangenen Daten hat das Datenverarbeitungsmodul 4 eine Verarbeitungseinheit 6, die insbesondere einen Mikroprozessor und einen Speicher aufweist.

Bei einer nicht dargestellten Ausführungsform ist das Datenverarbeitungsmodul 4 in eine Leseeinheit zum Einlesen der Sensordaten und in eine Datenverarbeitungseinheit zum Auslesen von diesen unterteilt.

Zur Erfassung von Umgebungsparametern ist in das Datenverarbeitungsmodul 4 mindestens ein externer Sensor 7 integriert. Mit dem externen Sensor 7 können zum Beispiel der Umgebungsluftdruck und/oder die Umgebungstemperatur zur Kalibrierung des Sensorsystems 1 gemessen werden.

Über eine Signalleitung 8 steht das Datenverarbeitungsmodul 4 mit einer Darstellungseinrichtung 9, zum Beispiel einem PC, einem Laptop oder einem PDA, in Verbindung. Bei einer nicht dargestellten Ausführungsform kann die Darstellungseinrichtung 9 auch in das Datenverarbeitungsmodul 4 oder in die Datenverarbeitungseinheit von diesem integriert sein. Mit Hilfe der Darstellungseinrichtung 9 werden die vom Datenverarbeitungsmodul 4 weitergeleiteten Daten visualisiert und gegebenenfalls noch nachbearbeitet.

Mit dem mindestens einen Sensor 2, dem Datenverarbeitungsmodul 4 und der Darstellungseinrichtung 9 ist das Sensorsystem 1 bei Verwendung eines kabelgebundenen Sensors 2 voll funktionsfähig. Zur Einbindung von Telemetrie-Sensoren zur Messung mindestens eines der vorstehend gelisteten physiologischen Parameter des Patienten ist das Sensorsystem 1 erweitert. Hierzu umfasst das Sensorsystem 1 ein Erweiterungsmodul 10, welches über eine Schnittstelle 11 und eine Signalleitung 12 mit einer Schnittstelle 13 des Datenverarbeitungsmoduls 4 verbunden ist. Bei den Schnittstellen 11, 13 handelt es sich um eine Standard-Schnittstelle, insbesondere um eine RS232-Schnittstelle. Entsprechend den jeweiligen Komponenten des Datenverarbeitungsmoduls 4 weist auch das Erweiterungsmodul 10 eine Steuereinheit 14, eine Verarbeitungseinheit 15 und einen externen Sensor 16 auf.

Über eine Signalleitung 17 steht eine Übertragungseinheit 18 des Erweiterungsmoduls 10 mit einer Leseeinheit 19 von diesem in Verbindung. Bei den Signalleitungen 8, 12, 17 kann es sich um Signalkabel oder um Telemetriestrecken, das heißt um nicht kabelgebundene Signalverbindungen, handeln. Im Falle einer kabellosen Signalverbindung über die Signalleitungen 8, 12, 17 ist die Schnittstelle als Blue-Tooth-Schnittstelle ausgeführt.

Die Leseeinheit 19 dient zur Datenkommunikation des Erweiterungsmoduls 10 einerseits mit mindestens einem Telemetrie-Sensor 20 über eine kabellose erste Telemetriestrecke 21 und andererseits mit dem Sensor 2 über eine kabellose zweite Telemetriestrecke 22. Die Telemetriestrecken 21, 22 können insbesondere eine Blue-Tooth-Schnittstelle aufweisen.

Die Übertragungseinheit 18 dient zur Weiterleitung der von der Leseeinheit 19 empfangenen Sensordaten an das Datenverarbeitungsmodul 4. Die weitergeleiteten Sensordaten können in der Übertragungseinheit 18 schon vorverarbeitet oder gänzlich zur Darstellung in der Darstellungseinrichtung 9 aufbereitet werden. In diesem letzteren Fall dient das Datenverarbeitungsmodul 4 als reine Übertragungskomponente der Sensordaten von der Übertragungseinheit 18 auf die Darstellungseinrichtung 9. Zur Verarbeitung von Sensordaten in der Übertragungseinheit 18 dient die Verarbeitungseinheit 15.

Über das Erweiterungsmodul 10 ist zudem eine Steuerung des Sensors 2 beziehungsweise des Telemetrie-Sensors 20 möglich. Hierzu hat das Erweiterungsmodul 10 die in die Übertragungseinheit 18 integrierte Steuereinheit 14. Die Steuerdaten werden über die Signalleitung 17, die Leseeinheit 19 und die entsprechende Telemetriestrecke 22, 21 auf die Sensoren 2, 20 übertragen. Sowohl die Signalleitung 17 als auch die Telemetriestrecken 21, 22 gewährleisten eine bidirektionale Signalverbindung.

Zur Realisierung der Telemetrie-Strecken 21, 22 weisen die Sensoren 2, 20 einerseits und die Leseeinheit 19 andererseits die hierfür erforderlichen und an sich bekannten Komponenten auf. Die Sensoren 2, 20 sind mit einem Transponder ausgerüstet, der zum Beispiel eine Sendefrequenz von 13,56 MHz hat. Die Leseeinheit 19 ist mit entsprechenden Komponenten ausgerüstet, die mit dieser Frequenz arbeiten, nämlich einer Leseantenne und einem Empfänger.

Das Sensorsystem 1 wird folgendermaßen eingesetzt: Das für die Messung, Übertragung, Verarbeitung und Darstellung physiologischer Parameter, die über kabelgebundene Sensoren 2 gewonnen werden, ausgelegte Grundsystem mit dem Sensor 2, dem Datenverarbeitungsmodul 4 und der Darstellungseinrichtung 9 wird mit Hilfe des Erweiterungsmoduls 10 und dem mindestens einen Telemetrie-Sensor 20 zum Sensorsystem 1 erweitert. Nunmehr können mit Hilfe des Sensorsystems 1 je nach Bedarf Sensordaten kabelgebundener und/oder kabellos angebundener Sensoren 2, 20 gemessen, übertragen, verarbeitet und dargestellt werden. Das Grundsystem ist nach dem Ausbau zum Sensorsystem 1 daher in seiner Funktionalität deutlich erweitert. Natürlich ist es möglich, das Sensorsystem 1 zu betreiben, ohne einen einzigen kabelgebundenen Sensor 2 einzusetzen, was die Bewegungsfreiheit des Patienten erhöht.

## Patentansprüche

1. Sensorsystem (1) zur Messung, Übertragung, Verarbeitung und Darstellung von physiologischen Parametern, umfassend ein Grund-Sensorsystem
- mit mindestens einem Sensor (2) zur Messung mindestens eines physiologischen Parameters,
- mit mindestens einem Datenverarbeitungsmodul (4),
- mit mindestens einer Darstellungseinrichtung (9), welche mit dem Datenverarbeitungsmodul (4) in Signalverbindung steht,
das Datenverarbeitungsmodul (4) mit dem Sensor (2) des Grund-Sensorsystems über ein Signalkabel (3) in Signalverbindung steht, zur Erweiterung der ausschließlich kabellosen Kommunikation des Grund-Sensorsystems ein Erweiterungsmodul (10) zur Übertragung von Telemetrie-Daten vorgesehen ist, welches
- über eine am Datenverarbeitungsmodul (4) vorgesehene Schnittstelle (13) mit diesem verbindbar ist, und
- über eine kabellose Telemetriestrecke (21, 22) mit dem mindestens einen Sensor (2) oder mindestens einem zusätzlichen Telemetrie-Sensor (20) zur Messung mindestens eines physiologischen Parameters in Signalverbindung steht,
**dadurch gekennzeichnet, dass**
ein mit Hilfe des Erweiterungsmoduls (10) dem Datenverarbeitungs-modul (4) durch Emulation vorgespiegelter, kabelgebundener Sensor vorgesehen ist.

2. Sensorsystem nach Anspruch 1, **dadurch gekennzeichnet, dass** das Erweiterungsmodul (10) eine Verarbeitungseinheit (15) zur Verarbeitung der vom Sensor (2, 20) empfangenen Daten aufweist.

3. Sensorsystem nach Anspruch 1 oder 2, **dadurch gekennzeichnet, dass** das Erweiterungsmodul (10) eine Leseeinheit (19) zur Datenkommunikation über die Telemetriestrecke (21, 22) und eine hiervon separate, mit dem Datenverarbeitungsmodul (4) verbundene Übertragungseinheit (18) aufweist.

4. Sensorsystem nach Anspruch 3, **dadurch gekennzeichnet, dass** die Übertragungseinheit (18) mit der Leseeinheit (19) über eine kabellose Telemetriestrecke verbunden ist.

5. Sensorsystem nach einem der Ansprüche 1 bis 4, **dadurch gekennzeichnet, dass** das Datenverarbeitungsmodul (4) und/oder das Erweiterungsmodul (19) über eine bidirektionale Signalverbindung (3, 21, 22) mit dem mindestens einen Sensor (2) oder dem mindestens einen zusätzlichen Sensor (20) verbunden ist.

6. Sensorsystem nach einem der Ansprüche 1 bis 5, **dadurch gekennzeichnet, dass** das Datenverarbeitungsmodul (4) und/oder das Erweiterungsmodul (10) eine Steuereinheit (5, 14) zur Sensorsteuerung aufweisen.

7. Sensorsystem nach einem der Ansprüche 1 bis 6, **dadurch gekennzeichnet, dass** der mindestens eine Sensor (2) oder der mindestens eine zusätzliche Sensor (20) mindestens einen der folgenden physiologischen Parameter misst:
- Hirndruck,
- Sauerstoffgehalt von Körperflüssigkeit beziehungsweise -gewebe,
- CO₂-Gehalt von Körperflüssigkeit beziehungsweise -gewebe,
- pH-Wert von Körperflüssigkeit,
- Körpertemperatur,
- Blutzuckergehalt,
- Blutfluss.

8. Sensorsystem nach einem der Ansprüche 1 bis 7, **gekennzeichnet durch** mindestens einen weiteren externen Sensor (7, 16), der mindestens einen der folgenden Umgebungsparameter misst:
- Luftdruck,
- Temperatur.

9. Sensorsystem nach Anspruch 8, **dadurch gekennzeichnet, dass** der externe Sensor (7, 16) in das Datenverarbeitungsmodul (4) und/oder das Erweiterungsmodul (10) integriert ist.

10. Sensorsystem nach einem der Ansprüche 1 bis 9, **dadurch gekennzeichnet, dass** die Telemetriestrecke (21, 22) eine Blue-Tooth-Schnittstelle aufweist.

## Claims

1. Sensor system (1) for measuring, transmitting, processing and representing physiological parameters, comprising a basic sensor system
- with at least one sensor (2) for measuring at least one physiological parameter,
- with at least one data processing module (4),
- with at least one display device (9), which is in a signal connection with the data processing module (4),
wherein the data processing module (4) is in signal connection with the sensor (2) of the basic sensor system via a signal cable (3), an expansion module (10) for transmitting telemetry data is provided for expanding the exclusively cable-free communication of the basic sensor system, which
- can be connected to the data processing module (4) via an interface (13) provided thereon, and
- is in a signal connection via a cable-free telemetry line (21, 22) with the at least one sensor (2) or at least one additional telemetry sensor (20) for measuring at least one physiological parameter,
**characterized in that**
a cable-connected sensor is provided, being simulated to the data processing module (4) by means of emulation with the aid of the expansion module (10).

2. Sensor system according to claim 1, **characterised in that** the expansion module (10) has a processing unit (15) for processing the data received from the sensor (2, 20).

3. Sensor system according to claim 1 or 2, **characterised in that** the expansion module (10) has a reader unit (19) for data communication via the telemetry line (21, 22) and a transmission unit (18) separate therefrom and connected to the data processing module (4).

4. Sensor system according to claim 3, **characterised in that** the transmission unit (18) is connected to the reader unit (19) via a cable-free telemetry line.

5. Sensor system according to any one of claims 1 to 4, **characterised in that** the data processing module (4) and/or the expansion module (19) is/are connected via a bidirectional signal link (3, 21, 22) to the at least one sensor (2) or to the at least one additional sensor (20).

6. Sensor system according to any one of claims 1 to 5, **characterised in that** the data processing module (4) and/or the expansion module (10) has/have a control unit (5, 14) for controlling the sensor.

7. Sensor system according to any one of claims 1 to 6, **characterised in that** the at least one sensor (2) or the at least one additional sensor (20) measures at least one of the following physiological parameters:
- pressure on the brain,
- oxygen content of body fluid or body tissue,
- CO₂ content of body fluid or body tissue,
- pH value of body fluid,
- body temperature,
- blood sugar content,
- blood flow.

8. Sensor system according to any one of claims 1 to 7, **characterised by** at least one further external sensor (7, 16) which measures at least one of the following ambient parameters:
- air pressure,
- temperature.

9. Sensor system according to claim 8, **characterised in that** the external sensor (7, 16) is integrated into the data processing module (4) and/or the expansion module (10).

10. Sensor system according to any one of claims 1 to 9, **characterised in that** the telemetry line (21, 22) has a Bluetooth interface.

## Revendications

1. Système à capteurs (1) servant à mesurer, à transmettre, à traiter et à afficher des paramètres physiologiques, comprenant un système à capteurs de base pourvu
- d'au moins un capteur (2) servant à mesurer au moins un paramètre physiologique,
- d'au moins un module de traitement de données (4),
- d'au moins un dispositif d'affichage (9), qui est en liaison de communication par signaux avec le module de traitement des données (4),
le module de traitement des données (4) étant en liaison de communication par signaux avec le capteur (2) du système à capteurs de base par l'intermédiaire d'un câble de signaux (3), un module d'extension (10) servant à transmettre des données de télémétrie étant prévu afin d'étendre la communication exclusivement sans fil du système à capteurs de base, lequel module d'extension
- peut être relié au module de traitement des données (4) par l'intermédiaire d'une interface (13) prévue au niveau dudit module, et
- qui est en liaison de communication par signaux avec le capteur (2) au moins au nombre de un ou avec au moins un capteur de télémétrie (20) supplémentaire servant à mesurer au moins un paramètre physiologique par l'intermédiaire d'une voie de télémétrie (21, 22) sans fil,
**caractérisé en ce**
**qu'**est prévu un capteur avec connectivité filaire, reproduit par émulation à l'aide du module d'extension (10) en amont du module de traitement des données (4).

2. Système à capteurs selon la revendication 1, **caractérisé en ce que** le module d'extension (10) présente une unité de traitement (15) servant à traiter les données reçues par le capteur (2, 20).

3. Système à capteurs selon la revendication 1 ou 2, **caractérisé en ce que** le module d'extension (10) présente une unité de lecture (19) servant à la communication de données par l'intermédiaire d'une voie de télémétrie (21, 22) et une unité de transmission (18) isolée de cette dernière, reliée au module de traitement des données (4).

4. Système à capteurs selon la revendication 3, **caractérisé en ce que** l'unité de transmission (18) est reliée à l'unité de lecture (19) par l'intermédiaire d'une voie de télémétrie sans fil.

5. Système à capteurs selon l'une quelconque des revendications 1 à 4, **caractérisé en ce que** le module de traitement des données (4) et/ou le module d'extension (19) est relié au capteur (2) au moins au nombre de un ou au capteur supplémentaire (20) au moins au nombre de un par l'intermédiaire d'une liaison de communication par signaux (3, 21, 22) bidirectionnelle.

6. Système à capteurs selon l'une quelconque des revendications 1 à 5, **caractérisé en ce que** le module de traitement des données (4) et/ou le module d'extension (10) présentent une unité de commande (5, 14) servant à la commande de capteur.

7. Système à capteurs selon l'une quelconque des revendications 1 à 6, **caractérisé en ce que** le capteur (2) au moins au nombre de un ou le capteur (20) supplémentaire au moins au nombre de un mesure au moins un des paramètres physiologiques suivants :
- la pression intracrânienne ;
- la teneur en oxygène d'un liquide corporel ou de tissus corporels ;
- la teneur en CO₂ d'un liquide corporel ou de tissus corporels ;
- la valeur pH d'un liquide corporel ;
- la température corporelle ;
- la glycémie ;
- le débit sanguin.

8. Système à capteurs selon l'une quelconque des revendications 1 à 7, **caractérisé par** au moins un autre capteur externe (7, 16), qui mesure au moins un des paramètres environnementaux suivants :
- la pression atmosphérique ;
- la température.

9. Système à capteurs selon la revendication 8, **caractérisé en ce que** le capteur (7, 16) externe est intégré dans le module de traitement des données (4) et/ou dans le module d'extension (10).

10. Système à capteurs selon l'une quelconque des revendications 1 à 9, **caractérisé en ce que** la voie de télémétrie (21, 22) présente une interface Bluetooth.
